# EUROPEAN PATENT APPLICATION

(11) **EP 3 514 182 A1**
(43) Date of publication of application: **24.07.2019**
(21) Application number: 17850805.7
(22) Date of filing: 08.09.2017
(51) Int. Cl.: C08B 30/18, A61K 31/724, A61P 31/04, C07H 19/056

(54) **SUGAR DERIVATIVE OR SALT THEREOF, AND ANTIBACTERIAL AGENT OR ANTIBACTERIAL ACTIVITY ENHANCER USING SAME**

(30) Priority: 13.09.2016 JP 2016178594
(71) Applicant: Nagoya Institute of Technology, 29 Aza Kiichi Gokiso-cho Showa-ku Nagoya-shi Aichi 466-0061 (JP)
(72) Inventor: YAMAMURA Hatsuo, Nagoya-shi Aichi 466-0061 (JP); MIYAGAWA Atsushi, Nagoya-shi Aichi 466-0061 (JP)
(74) Representative: Winter, Brandl, Fürniss, Hübner, Röss, Kaiser, Polte - Partnerschaft mbB
(86) International application number: PCT/JP2017/032426
(87) International publication number: WO 2018/051903

(57) **Abstract**

[Problem] Provided are: a compound having excellent antimicrobial activity, and a compound having action for enhancing the antimicrobial activity of another antimicrobial agent.

[Solution] Disclosed is a saccharide derivative represented by Chemical Formula (1) or (2), or a salt thereof. This compound includes units of a monosaccharide linked to each other in a chainlike or cyclic manner through glycosidic bonding and has antimicrobial action. In the formulae, R¹ represents an amino-containing structure; and n represents an integer of 3 or more. Also disclosed is a saccharide derivative represented by Chemical Formula (5) or (6), or a salt thereof. This compound includes units of a monosaccharide linked to each other in a chainlike or cyclic manner through glycosidic bonding and has antimicrobial activity enhancing action. In the formulae, R³ represents an amino-containing structure; R⁴ is selected from acetyl and propanoyl; and n represents an integer of 3 or more.

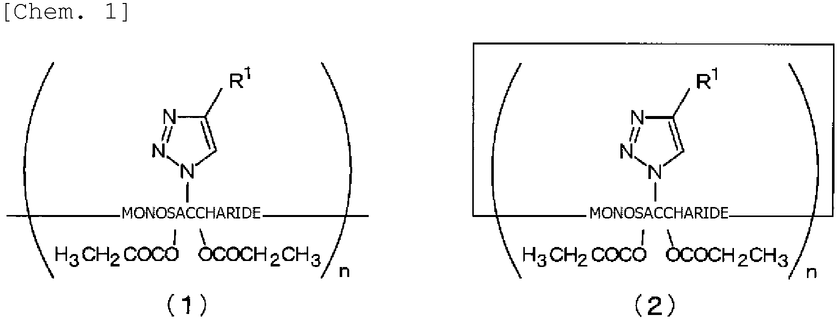

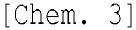

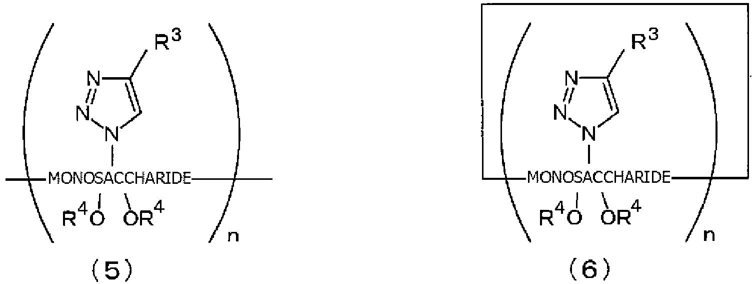

## Description

### Technical Field

The present invention relates to sugar derivatives (saccharide derivatives) or salts thereof, and antimicrobial agents or antimicrobial activity enhancers containing the same.

### Background Art

Since the discovery of penicillin, various antibiotics and synthetic antimicrobial drugs have been developed and used for the therapy of infectious diseases. However, strong demands are made for the development of novel antimicrobial drugs, because multidrug-resistant microorganisms, represented by methicillin resistant Staphylococcus Aureus (MRSA), have been seriously increased, where the multidrug-resistant microorganisms are resistant to approximately most of existing antimicrobial drugs.

Existing antimicrobial substances that generally less cause drug resistance include polymyxin B and other substances offering antimicrobial activity due to the membrane-active mechanism. These substances are, however, natural products having complicated structures and are not easy to produce artificially and, consequently, to improve so as to have better activity. As a possible solution to this disadvantage, artificial antimicrobial substances have been synthesized and, for example, cyclodextrins having specific chemical structures are chemically modified and used as antimicrobial agents (typically see Patent Literature (PTL) 1, 2, and 3).

### Citation List

### Patent Literature

PTL 1: PCT International Publication Number WO2006/075580 (Japanese Patent No. 5098015)
PTL 2: PCT International Publication Number WO2006/083678
PTL 3: Japanese Unexamined Patent Application Publication No. 2013-177477

### Summary of Invention

### Technical Problem

However, demands are still made for better antimicrobial activity and/or better antimicrobial activity enhancing action as compared with the substances according to the conventional technology, so as to provide for changes of bacteria typically in drug resistance. The present invention has been made under these circumstances and has an object to specify, in the chemical formula, a compound that has better antimicrobial activity and/or better activity of enhancing the antimicrobial activity of another antimicrobial agent, as compared with the substances according to the conventional technology (such as the compounds disclosed in PTL 3) and to provide, using the compound, an antimicrobial agent and an agent for enhancing the activity of another antimicrobial agent.

### Solution to Problem

The present invention provides, in a first embodiment, a saccharide derivative represented by Chemical Formula (1) or (2), or a salt thereof. The saccharide derivative includes units of a monosaccharide linked to each other in a chainlike or cyclic manner through glycosidic bonding and has antimicrobial action.

Chemical Formulae (1) and (2) are expressed as follows, in which R¹ represents an amino-containing structure, where R¹ is bonded to the specified triazole replacing a primary hydroxy group of the monosaccharide. The propanoyl groups each replace a hydrogen atom of a secondary hydroxy group of the monosaccharide. The number n represents an integer of 3 or more.

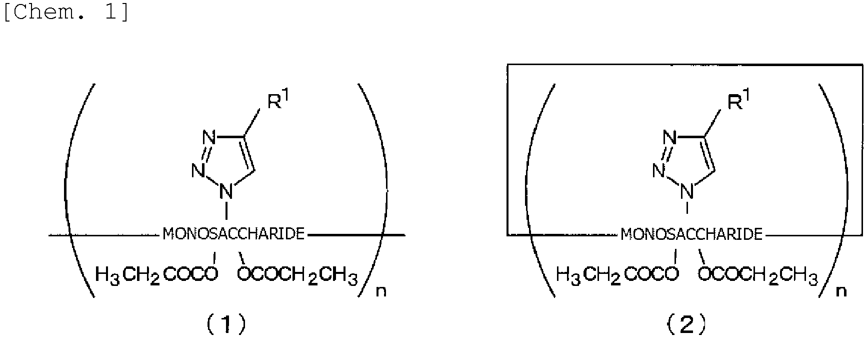

The present invention also provides, in a second embodiment, a saccharide derivative represented by Chemical Formula (3) or (4), or a salt thereof. The saccharide derivative includes units of a monosaccharide linked to each other in a chainlike or cyclic manner through glycosidic bonding and has antimicrobial action. Chemical Formulae (3) and (4) are expressed as follows, in which R² represents an alkylene group, where R²NH₂ is bonded to the triazole replacing a primary hydroxy group of the monosaccharide. The propanoyl groups each replace a hydrogen atom of a secondary hydroxy group of the monosaccharide. The number n represents an integer of 3 or more.

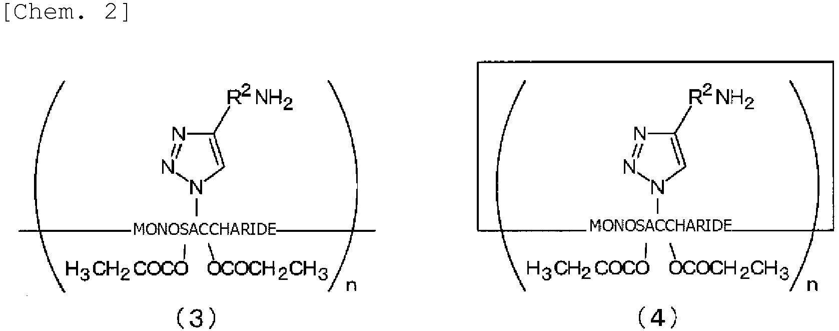

In the saccharide derivative or a salt thereof according to the second embodiment of the present invention, R² in Chemical Formula (3) or (4) may be a methylene group (third embodiment) .

In the saccharide derivative or a salt thereof according to any one of the first to third embodiments, the monosaccharide in the chemical formula may be glucose (fourth embodiment).

In the saccharide derivative or a salt thereof according to any one of the first to third embodiments, the monosaccharide in the chemical formula may be glucose, and the saccharide derivative may be an oligosaccharide including units of the glucose linked to each other in a cyclic manner (fifth embodiment).

The present invention provides, in a sixth embodiment, an antimicrobial agent that contains, as an active ingredient, the saccharide derivative or a salt thereof according to any one of the first to fifth embodiments.

The present invention provides, in a seventh embodiment, a saccharide derivative represented by Chemical Formula (5) or (6), or a salt thereof. This compound includes units of a monosaccharide linked to each other in a chainlike or cyclic manner through glycosidic bonding and has antimicrobial activity enhancing action. Chemical Formulae (5) and (6) are expressed as follows, in which R³ represents an amino-containing structure, where R³ is bonded to the specified triazole replacing a primary hydroxy group of the monosaccharide, and where the amino-containing structure is represented typically by an aminoalkyl group. R⁴ is, independently in each occurrence, selected from acetyl and propanoyl and replaces a hydrogen atom of a secondary hydroxy group of the monosaccharide. The number n represents an integer of 3 or more.

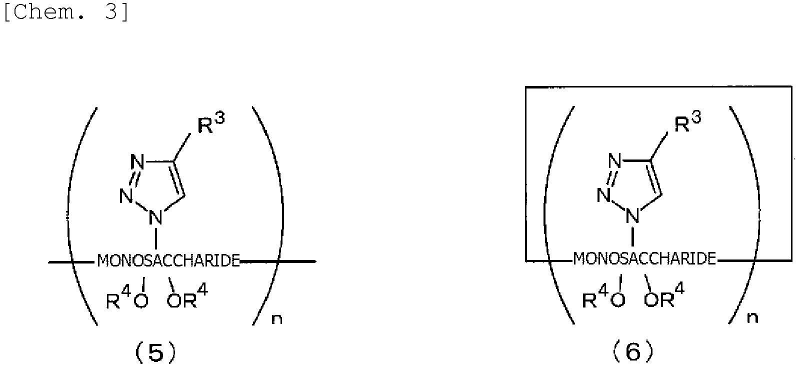

The present invention provides, in an eighth embodiment, a saccharide derivative represented by Chemical Formula (7) or (8), or a salt thereof. This compound includes units of a monosaccharide linked to each other in a chainlike or cyclic manner through glycosidic bonding and has antimicrobial action and/or antimicrobial activity enhancing action. Chemical Formulae (7) and (8) are expressed as follows, in which R⁵ represents an alkylene group, where R⁵NH₂ is bonded to the triazole replacing a primary hydroxy group of the monosaccharide. R⁶ is, independently in each occurrence, selected from acetyl and propanoyl and replaces a hydrogen atom of a secondary hydroxy group of the monosaccharide. The number n represents an integer of 3 or more.

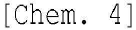

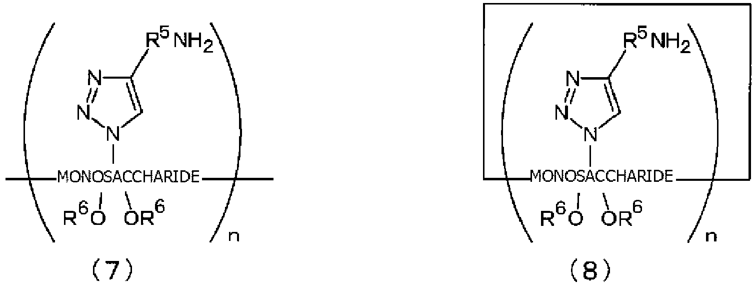

In the saccharide derivative or a salt thereof according to the eighth embodiment, R⁵ in Chemical Formula (7) or (8) may be a methylene group (ninth embodiment).

In the saccharide derivative or a salt thereof according to any one of the seventh to ninth embodiments, the monosaccharide in the chemical formula may be glucose (tenth embodiment).

In the saccharide derivative or a salt thereof according to any one of the seventh to ninth embodiments, the monosaccharide in the chemical formula may be glucose, and the saccharide derivative may be an oligosaccharide including units of the glucose linked to each other in a cyclic manner (eleventh embodiment).

The present invention also provides, in a twelfth embodiment, an antimicrobial activity enhancer that contains, as an active ingredient, the saccharide derivative or a salt thereof according to any one of the seventh to eleventh embodiments. Advantageous Effects of Invention

The present invention has been made under the conventional circumstances and can specify, in chemical formula, compounds that have better activities as antimicrobial agents and as enhancers for enhancing the activity of another antimicrobial agent, as compared with the those according to the conventional technology. Thus, the present invention can provide antimicrobial agents and antimicrobial activity enhancers containing the compounds, where the antimicrobial activity enhancers enhance the activity of another antimicrobial agent.

The first embodiment of the present invention can advantageously specify the chemical formula and provide, as an antimicrobial saccharide derivative or a salt thereof, a saccharide derivative of Chemical Formula (1) or (2), or a salt thereof. This compound includes units of a monosaccharide linked to each other in a chainlike or cyclic manner through glycosidic bonding, where, in the monosaccharide, an amino-containing structure-bonded triazole ring replaces the primary hydroxy group, and propanoyl groups are substituted on the secondary hydroxy groups.

The second embodiment of the present invention can advantageously specify, in chemical formula, and provide, as an antimicrobial saccharide derivative or a salt thereof, a saccharide derivative of Chemical Formula (3) or (4), or a salt thereof. This compound includes units of a monosaccharide linked to each other in a chainlike or cyclic manner through glycosidic bonding, where, of the monosaccharide, an aminoalkyl-bonded triazole ring replaces the primary hydroxy group, and propanoyl groups are substituted on the secondary hydroxy groups.

The third embodiment of the present invention can advantageously specify the chemical formula and provide, as an antimicrobial saccharide derivative or a salt thereof, the saccharide derivative or a salt thereof according to the second embodiment in which the aminoalkyl group is aminomethyl

The fourth embodiment of the present invention can advantageously specify the chemical formula and provide, as an antimicrobial saccharide derivative or a salt thereof, the saccharide derivative or a salt thereof according to any one of the first to third embodiments in which the monosaccharide is glucose.

The fifth embodiment of the present invention can advantageously specify the chemical formula and provide, as an antimicrobial saccharide derivative or a salt thereof, the saccharide derivative or a salt thereof according to any one of the first to fourth embodiments in which the monosaccharide is glucose, and the saccharide derivative is an oligosaccharide including units of the glucose linked to each other in a cyclic manner.

The sixth embodiment of the present invention can advantageously provide an antimicrobial agent that contains, as an active ingredient, the saccharide derivative or a salt thereof according to any one of the first to fifth embodiments, which is a compound having satisfactory antimicrobial action.

The seventh embodiment of the present invention can advantageously specify the chemical formula and provide, as an antimicrobial activity enhancing saccharide derivative or a salt thereof, the saccharide derivative represented by Chemical Formula (5) or (6), or a salt thereof. This compound includes units of a monosaccharide linked to each other in a chainlike or cyclic manner through glycosidic bonding, where, in the monosaccharide, an amino-containing structure-bonded triazole ring replaces the primary hydroxy group, and R⁴s each substituted on the secondary hydroxy group are selected from acetyl and propanoyl.

The eighth embodiment of the present invention can advantageously specify the chemical formula and provide, as an antimicrobial or antimicrobial activity enhancing saccharide derivative or a salt thereof, the saccharide derivative represented by Chemical Formula (7) or (8), or a salt thereof. This compound includes units of a monosaccharide linked to each other in a chainlike or cyclic manner through glycosidic bonding, where, in the monosaccharide, an aminoalkyl-bonded triazole ring replaces the primary hydroxy group, and R⁴s substituted on the secondary hydroxy groups are selected from acetyl and propanoyl.

The ninth embodiment of the present invention can advantageously specify the chemical formula and provide, as an antimicrobial activity enhancing saccharide derivative or a salt thereof, the saccharide derivative or a salt thereof according to the eighth embodiment in which the aminoalkyl group is aminomethyl.

The tenth embodiment of the present invention can advantageously specify the chemical formula and provide, as an antimicrobial activity enhancing saccharide derivative or a salt thereof, the saccharide derivative or a salt thereof according to any one of the seventh to ninth embodiments in which the monosaccharide is glucose.

The eleventh embodiment of the present invention can advantageously specify the chemical formula and provide, as an antimicrobial activity enhancing saccharide derivative or a salt thereof, the saccharide derivative or a salt thereof according to any one of the seventh to ninth embodiments in which the monosaccharide is glucose, and the saccharide derivative is an oligosaccharide including units of the glucose linked to each other in a cyclic manner.

The twelfth embodiment of the present invention can advantageously provide an antimicrobial activity enhancer that contains, as an active ingredient, the saccharide derivative or a salt thereof according to any one of the seventh to eleventh embodiments, as a compound having satisfactory antimicrobial activity enhancing action.

As described above, the present invention can achieve the objects by providing saccharide derivatives that have better antimicrobial activity and/or better antimicrobial activity enhancing action as compared with those according to the conventional technology.

### Description of Embodiments

Some embodiments of the present invention will be illustrated below. It should be noted, however, that the embodiments are never construed to limit the scope of the present invention; and various changes, modifications, and improvements can be made therein without departing from the spirit and scope of the present invention.

Initially, the inventors of the present invention focused on the cyclodextrin chemical structure indicated below, thought up the idea of the possibility that this chemical structure is usable in a novel antimicrobial substance, and conceived the following molecular design.

A cyclodextrin is a cyclic saccharide (cyclic sugar) that includes units of monosaccharide glucose linked to each other in a cyclic manner and has a diameter of about 1 nanometer. One including eight glucose units is called gamma-cyclodextrin (see Chemical Formula (9) below). Of glucose, the primary hydroxy group at the 6-position is oriented toward one of two openings of the cyclic structure, and the secondary hydroxy groups at the 2-position and the 3-position are oriented to the other opening. The primary and secondary hydroxy groups differ from each other in reactivity, and can undergo chemical modification selectively.

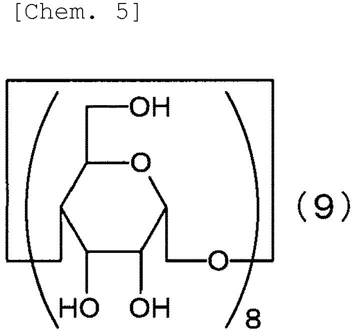

The cyclic saccharide cyclodextrin represented by Chemical Formula (9) has analogues different in number of constitutive glucose units. Ones including five or more glucose units are known. On the other hand, two or more glucose units are linked to each other in a chainlike manner to form oligosaccharides and polysaccharides. These oligosaccharides and polysaccharides include glucans typified by amylose. In addition, there are polysaccharides each including units of a monosaccharide that is a glucose isomer, such as mannans each including mannose as a constitutional repeating structure. These also have hydroxy groups differing in reactivity on the constitutive repeating structure monosaccharide and can selectively undergo chemical modification.

Polymyxin B, whose structure is represented by Chemical Formula (10) below, is a peptide having antimicrobial activity on some bacteria. This is a naturally-occurring cyclic peptide. Polymyxin B injures the membrane structure of a bacterium to offer antimicrobial activity. A non-limiting example of the structural feature that can cause the injury is that this compound has amino acid moieties each having a cationic amino group.

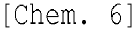

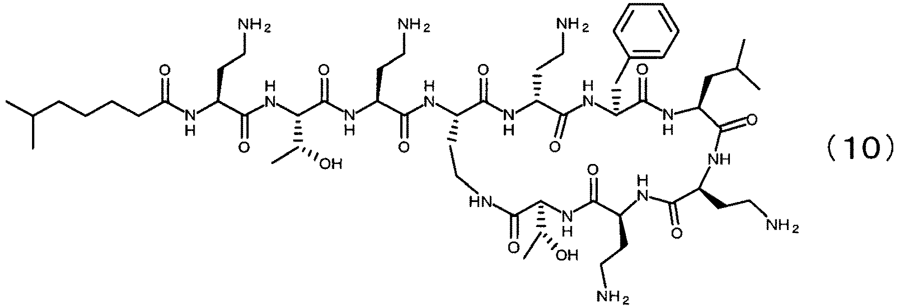

Under these circumstances, the inventors conceived the integration of amino acid moieties each having a cationic amino group on a cyclodextrin, so as to allow the cyclodextrin to actually have antimicrobial activity against bacteria.

To integrate amino acid moieties, the inventors studied the technique of introducing the amino acid moieties through click chemistry using azide groups introduced onto the cyclodextrin. The click chemistry refers to a reaction having features as follows:
(1) high versatility;
(2) high efficiency and high yield;
(3) no by-products;
(4) stereospecificity;
(5) easy purification; and
(6) easy availability of starting materials and reagents.

The Huisgen reaction (see the following chemical reaction formula) is considered to be the most general reaction according to click chemistry. This reaction is the reaction of an azide with a terminal alkyne to form a triazole ring. The reaction, when catalyzed by copper, forms a 1,4-disubstituted isomer and proceeds at a significantly higher rate. The azide and the alkyne react moderately only with each other and are stable to nucleophiles and electrophiles that are commonly used under standard reaction conditions. The formed triazole ring is also a stable functional group. On the basis of the features as above, the Huisgen reaction is employed in a wide variety of fields.

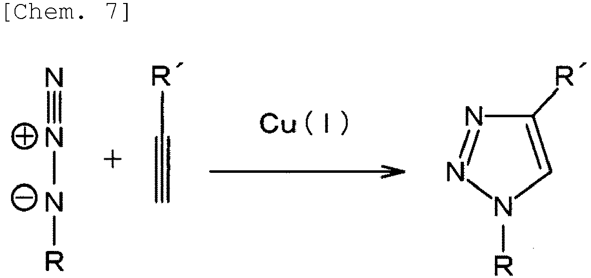

The inventors synthesized a compound by, into a cyclodextrin, introducing an amino-containing structure using the Huisgen reaction, and introducing specific acyl groups. The inventors found that the synthesized compound functions significantly more satisfactorily as an antimicrobial agent and/or an enhancer for enhancing the activity of another antimicrobial agent, as compared with known compounds. The present invention has been made on the basis of these findings.

Embodiments Relating to First to Twelfth Embodiments of Present Invention

The first embodiment of the present invention is a saccharide derivative represented by Chemical Formula (1) or (2), or a salt thereof. This compound includes units of a monosaccharide linked to each other in a chainlike or cyclic manner through glycosidic bonding and has antimicrobial action. Chemical Formulae (1) and (2) are expressed as follows, in which R¹ represents an amino-containing structure, where R¹ is bonded to the triazole ring replacing the primary hydroxy group of the monosaccharide. The amino-containing structure (R¹) is typically an aminoalkyl group containing a terminal amino group, or is an alkylaminoalkyl group containing an inlying amino group. The propanoyl groups each replace a hydrogen atom of a secondary hydroxy group of the monosaccharide. The number n represents an integer of 3 or more:

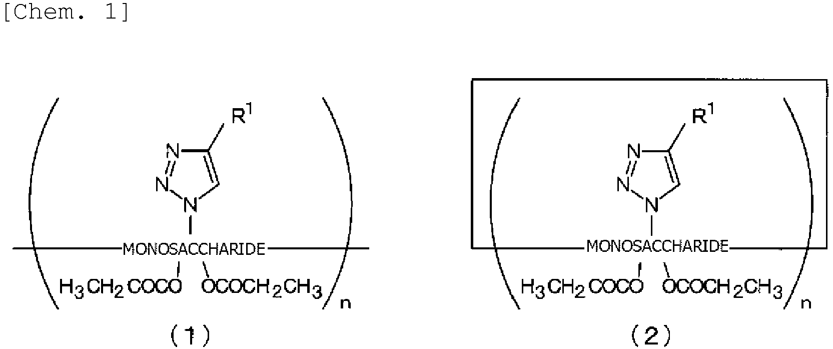

The second embodiment of the present invention is a saccharide derivative represented by Chemical Formula (3) or (4), or a salt thereof. This compound includes units of a monosaccharide linked to each other in a chainlike or cyclic manner through glycosidic bonding and has antimicrobial action. Chemical Formulae (3) and (4) are expressed as follows, in which R²NH₂ is bonded to the triazole replacing a primary hydroxy group of the monosaccharide. The propanoyl groups each replace a hydrogen atom of a secondary hydroxy group of the monosaccharide. The number n represents an integer of 3 or more. The second embodiment further specifies the chemical formula, by specifying R¹ in the first embodiment as R²NH₂, where R² is an alkylene group.

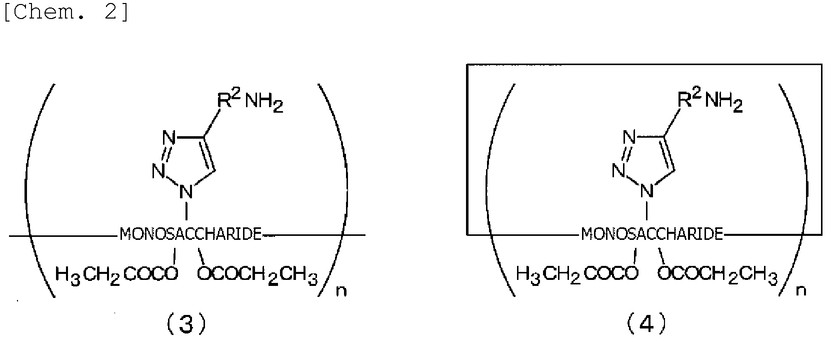

R² in the chemical formula is preferably a methylene group. This corresponds to the saccharide derivative or a salt thereof according to the third embodiment of the present invention.

The number n is set as a repetition number in an existing oligosaccharide or polysaccharide. For example, n can be 1000 or less; and is preferably from 5 to 100.

Non-limiting examples of the compound according to the present invention include saccharide derivatives whose monosaccharide unit is a hexose, which contains 6 carbon atoms, or salts thereof. Representative, but non-limiting examples of the hexose include glucose, mannose, fructose, and galactose.

Non-limiting examples of the compound according to the present invention also include saccharide derivatives or salts thereof, whose monosaccharide unit is glucose. These correspond to the saccharide derivative or a salt thereof according to the fourth embodiment of the present invention.

Non-limiting examples of the compound according to the present invention also include oligosaccharide derivatives or salts thereof, whose monosaccharide unit is glucose, and which include glucose units linked to each other in a cyclic manner. More particularly, a non-limiting example of the compound is an oligosaccharide derivative or a salt thereof, in which n is 8, and which includes glucose units linked to each other in a cyclic manner. This corresponds to the saccharide derivative or a salt thereof according to the fifth embodiment of the present invention.

An antimicrobial agent according to the present invention contains, as an active ingredient, the saccharide derivative or a salt thereof. As used herein, the phrase "to contain as an active ingredient" refers to not only the case where the saccharide derivative or a salt thereof constitutes the entire structure of the antimicrobial agent, but also the case where the saccharide derivative or a salt thereof constitutes a part of the structure of the antimicrobial agent. This corresponds to the antimicrobial agent according to the sixth embodiment of the present invention.

The seventh embodiment of the present invention is a saccharide derivative represented by Chemical Formula (5) or (6), or a salt thereof. This compound includes units of a monosaccharide linked to each other in a chainlike or cyclic manner through glycosidic bonding and has antimicrobial activity enhancing action. In the formulae, R³ is an amino-containing structure, where R³ is bonded to the triazole ring replacing a primary hydroxy group of the monosaccharide. The amino-containing structure (R³) is typically an aminoalkyl group containing a terminal amino group, or an alkylaminoalkyl group containing an internal amino group. R⁴ is, independently in each occurrence, selected from acetyl and propanoyl and replaces a hydrogen atom of a secondary hydroxy group of the monosaccharide. The number n represents an integer of 3 or more.

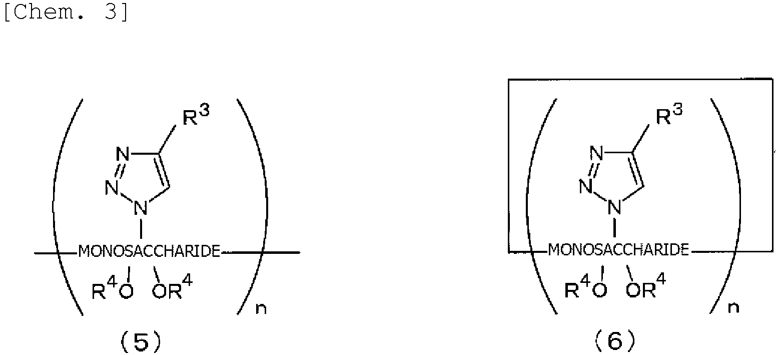

The eighth embodiment of the present invention is a saccharide derivative represented by Chemical Formula (7) or (8), or a salt thereof. This compound includes units of a monosaccharide linked to each other in a chainlike or cyclic manner through glycosidic bonding and has antimicrobial action and/or antimicrobial activity enhancing action. In the chemical formulae, R⁵NH₂ is bonded to the triazole replacing a primary hydroxy group of the monosaccharide. R⁶ is, independently in each occurrence, selected from acetyl and propanoyl and replaces a hydrogen atom of a secondary hydroxy group of the monosaccharide. The number n represents an integer of 3 or more. The eighth embodiment of the present invention further specifies the chemical formula, by specifying R³ in the seventh embodiment as R⁵NH₂, where R⁵ is an alkylene group.

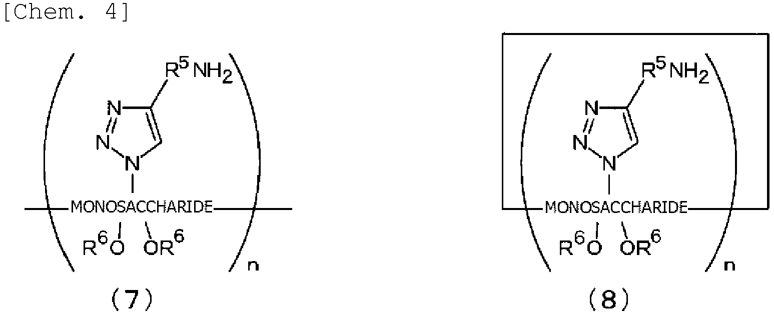

R⁵ herein is preferably a methylene group. This corresponds to the saccharide derivative or a salt thereof according to the ninth embodiment of the present invention.

The number n is determined as a repetition number in an existing oligosaccharide or polysaccharide. The number n can be typically 1000 or less, and is preferably from 5 to 100.

Non-limiting examples of the compound according to the present invention include saccharide derivatives or salts thereof, whose monosaccharide unit is a hexose, which contains 6 carbon atoms. Non-limiting examples of the hexose include glucose, mannose, fructose, and galactose.

A non-limiting example of the compound according to the present invention is a saccharide derivative or a salt thereof, whose monosaccharide unit is glucose. This corresponds to the saccharide derivative or a salt thereof according to the tenth embodiment of the present invention.

A non-limiting example of the compound according to the present invention is also an oligosaccharide derivative or a salt thereof, whose monosaccharide unit is glucose, and which includes units of the glucose linked to each other in a cyclic manner. This corresponds to the saccharide derivative or a salt thereof according to the eleventh embodiment of the present invention. A more specific, but non-limiting example of the compound is an oligosaccharide derivative or a salt thereof, in which n is 8, and eight units of glucose are linked to each other in a cyclic manner.

An antimicrobial activity enhancer according to the present invention contains, as an active ingredient, the saccharide derivative or a salt thereof. As used herein, the phrase "to contain as an active ingredient" refers to not only the case where the saccharide derivative or a salt thereof constitutes the entire structure of the antimicrobial activity enhancer, but also the case where the saccharide derivative or a salt thereof constitutes a part of the structure of the antimicrobial activity enhancer. This corresponds to the antimicrobial activity enhancer according to the twelfth embodiment of the present invention.

### Molecular Design

The molecular designing of the antimicrobial substances according to the present invention was performed on the basis of the following concept, so as to synthesize the substances.

The presence of two or more amino acid moieties each having an amino group in a peptide is important so as to damage the bacterial membrane to thereby offer antimicrobial activity (namely, antimicrobial action) against bacteria. On the basis of this, amino groups are integrated on a cyclodextrin. To achieve this, a hydrophilic, cationic moiety is constituted by linking an amino-containing structure through a triazole moiety to the glucose moiety (glucose unit) of the cyclodextrin. Upon bacterial membrane injury, this moiety is initially bonded to the anionic phosphoric acid moiety of the bacterial membrane and damages the bacterial membrane. In addition, a hydrophobic moiety, when present in the compound, interacts with an aliphatic chain of the bacterial membrane. The antimicrobial activity or antimicrobial activity enhancing ability does not exhibit unless the balance between the cationic moiety and the hydrophobic moiety is achieved. Ones that enable the balance are propanoyl groups each replacing a hydrogen atom of a secondary hydroxy group and serving as a hydrophobic moiety. As described above, it is important that a cyclodextrin derivative includes glucose units containing both an amino-containing structure introduced through the triazole moiety and a hydrophobic moiety or moieties, so as to function as an antimicrobial substance. This corresponds to the saccharide derivative or a salt thereof, and the operations of them, according to the fifth embodiment of the present invention. It is also speculated that an oligosaccharide and a polysaccharide exhibit the antimicrobial activity by introducing such a structure onto glucose units of a saccharide by a similar procedure as above, where the oligosaccharide includes units of glucose linked to each other in a chainlike manner, and the polysaccharide includes units of glucose linked to each other in a chainlike or cyclic manner. This corresponds to the saccharide derivative or a salt thereof, and the operations of them, according to the fourth embodiment of the present invention.

Examples of glucose isomers include monosaccharide hexoses typified by mannose. These have carbons and hydroxy groups in numbers identical to those in glucose and offer hydrophilicity similar to that of glucose. There are oligosaccharides and polysaccharides including these linked to each other. Representative, but non-limiting example of these include mannans. It is speculated that these oligosaccharides and polysaccharides also exhibit antimicrobial activity by introducing such a structure on repetitive structure monosaccharide units by a similar procedure as above. This corresponds to the saccharide derivative or a salt thereof, and the operations of them, according to any one of the first to third embodiments of the present invention.

A substance that contains, as an active ingredient, the oligosaccharide or polysaccharide according to any one of the embodiments of the present invention functions as an antimicrobial agent. This corresponds to the antimicrobial agent according to the sixth embodiment of the present invention. As used herein, the phrase "to contain as an active ingredient" refers to not only the case where the oligosaccharide or polysaccharide derivative or a salt thereof constitutes the entire structure of the antimicrobial agent, but also the case where the compound constitutes a part of the structure.

On the other hand, when the hydrophobic moiety is acetyl or propanoyl replacing a hydrogen atom of a secondary hydroxy group, it is important that a cyclodextrin derivative contains glucose units containing both the hydrophobic moiety and an amino-containing structure introduced thereinto through a triazole moiety by the above procedure, so as to function as an antimicrobial activity enhancer. This is probably also true for oligosaccharides including glucose units linked to each other in a cyclic manner. The saccharide derivative or a salt thereof according to the eleventh embodiment of the present invention, and operations thereof, are as described above. It is also speculated that an oligosaccharide or a polysaccharide functions as an antimicrobial activity enhancer by introducing such a structure into the glucose units of the saccharide by a procedure as above, where the oligosaccharide includes glucose units linked to each other in a chainlike manner, and the polysaccharide includes glucose units linked to each other in a chainlike or cyclic manner. The saccharide derivative or a salt thereof, and operations of them, according to the tenth embodiment of the present invention are as described above.

Examples of glucose isomers include monosaccharide hexoses typified by mannose. These have carbons and hydroxy groups in numbers identical to those in glucose and offer hydrophilicity similar to that of glucose. There are oligosaccharides and polysaccharides including such hexose units linked to each other. Representative, but non-limiting example of them include mannans. It is speculated that these oligosaccharides and polysaccharides also exhibit antimicrobial activity enhancing ability by introducing such a structure onto the repetitive structure monosaccharide units by a similar procedure as above. This corresponds to the saccharide derivative or a salt thereof, and operations of them, according to any one of the seventh to ninth embodiments of the present invention.

A substance that contains, as an active ingredient, the oligosaccharide or polysaccharide derivative or a salt thereof according to any one of the embodiments functions as an antimicrobial activity enhancer. This corresponds to the antimicrobial activity enhancer according to the twelfth embodiment of the present invention. As used herein, the phrase "to contain as an active ingredient" refers to not only the case where the oligosaccharide or polysaccharide derivative or a salt thereof constitutes the entire structure of the antimicrobial agent or antimicrobial activity enhancer, but also the case where the compound constitutes a part of the structure.

### Examples

A compound (a), namely, a cyclodextrin derivative (a) represented by Chemical Formula (11) was synthetically prepared in Example 1, and was evaluated for antimicrobial activity and antimicrobial activity enhancing action in Examples 2 to 4. The compound (a) corresponds to a compound of General Formula (2) in which R¹ is CH₂NH₂, and n is 8.

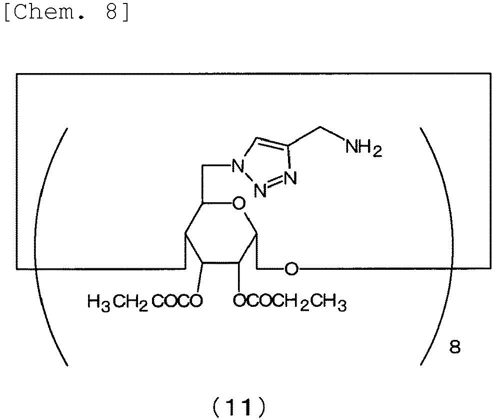

### Example 1

The way to synthetically prepare the cyclodextrin derivative (a) represented by Chemical Formula (11) will be described in detail below. Cyclodextrin octaazide (327.2 mg; 2.19 ×10⁻⁴ mol) was dissolved in pyridine (10.0 ml) and was combined with 85.2 mg (6.97 ×10⁻⁴ mol) of DMAP and a total of 5.4 ml (4.2 ×10⁻² mol) of propionic anhydride, followed by stirring. After 47 hours, the resulting solution was combined with methanol, subjected to distilling off under reduced pressure to remove the solvent, the residue was combined with 100 ml of ethyl acetate and was washed with water, hydrochloric acid, aqueous saturated sodium hydrogencarbonate solution, and saturated brine. The solvent was distilled off under reduced pressure, and the residue was purified using a normal-phase silica gel column (hexane/ethyl acetate). The target fraction was collected, from which the solvent was distilled off under reduced pressure. This gave 362.5 mg (1.51 ×10⁻⁴ mol, 69.3%) of a propanoate. An acetone solution (2.0 ml, 24.2 mg/ml, 2.02 ×10⁻⁵ mol) of the propanoate was combined with 400 µl (10.3 mg/ml, 1.64 ×10⁻⁵ mol) of an aqueous CuSO₄·5H₂O solution and a Boc-propargylamine DMSO solution (2.0 ml, 15.7 mg/ml, 2.02 ×10⁻⁴ mol), followed by stirring with microwave heating (100°C) for 30 minutes. The reaction mixture (solution) was combined with 50 ml of ethyl acetate, washed with an aqueous 5% EDTA disodium dihydrate solution, and dried over saturated brine and sodium sulfate. The solvent was distilled off under reduced pressure, and the residue was purified using a normal-phase silica gel column (methylene chloride/methanol). The target fraction was collected, from which the solvent was distilled off under reduced pressure. This gave 65.3 mg (1.80 ×10⁻⁵ mol, 89.0%) of a click reaction product. The click reaction product (42.1 mg, 1.16 ×10⁻⁵ mol) was combined with 2.0 ml of trifluoroacetic acid, followed by stirring at room temperature for 3.5 hours. The solution was subjected to distilling off under reduced pressure to remove the solvent, the residue was subjected to lyophilization and yielded 38.4 mg (1.03 ×10⁻⁵ mol, 88.5% as an octa(trifluoroacetate) salt) of the cyclodextrin derivative (a). This indicates that a salt of the saccharide derivative according to any one of the first to sixth embodiments can be produced.
¹H-NMR (400 MHz, DMSO-d₆) : δ = 1.03 (42H, OCOCH₂CH₃), 2.38-2.16 (28H, OCOCH₂CH₃), 3.61 (7H, H-4), 4.06-3.99 (14H, CH₂NH₃⁺), 4.39 (7H, H-5), 4.72-4.65 (21H, H-2,6), 5.35-5.29 (14H, H-1,3), 8.11 (7H, CH-triazole), 8.39 (21H, CH₂NH₃⁺),
¹³C-NMR (125 MHz, DMSO-d₆) : δ = 173.18-172.63 (C=O), 140.32 (CH-triazole), 126.19 (C-triazole), 96.00 (C-1), 76.21 (C-4), 69.63 (C-2, 3, 5), 49.52 (C-6), 33.88 (CH₂NH₃⁺), 26.64 (CH₂CH₃), 8.69-8.56 (CH₂CH₃)
MS: [M+Na]⁺ Calcd. for C₁₂₀H₁₇₆N₃₂NaO₄₈: 2856.2212, Found: 2856.5737
Anal: Calcd. for C₁₂₆N₃₂O₄₈+ 16H₂O+8CF₃COOH: C 40.48, H 5.40, N 11.11.
Found: C 40.63, H 5.30, N 10.84

### Example 2

The cyclodextrin derivative (a) synthetically prepared according to Example 1 was evaluated for antimicrobial activity on the basis of minimum inhibitory concentration. The evaluation was performed using Gram-positive bacterium *Bacillus subtilis,* Gram-negative bacterium *Escherichia coli,* Gram-positive bacterium *Staphylococcus aureus,* and Gram-negative bacteria *Salmonella typhimurium* and *Pseudomonas aeruginosa.*

The evaluation was performed by a procedure as follows. Specifically, before testing, each of the bacteria were incubated on a minimal salt medium combined with common bacteria-use dried broth or polypeptone and reached a logarithmic growth phase. In the testing, the bacterium was incubated at 37°C for 20 hours with a test compound, and the concentration at which the test compound inhibited the growth of the bacterium was defined as the minimum inhibitory concentration. The testing incubation was performed at an initial bacterial concentration of 1 x10⁴ cells per milliliter. The determined minimum inhibitory concentrations (MICs) are given in Table 1.

**[Table 1]**

| | Minimum Inhibitory Concentration (in µg/mL) | | | | |
|---|---|---|---|---|---|
| | *Bacillus subtilis* | *Staphylococcus aureus* | *Escherichia coli* | *Salmonella typhimurium* | *Pseudomonas aeruginosa* |
| Compound (a) (Example 1) | 8 | 32 | 16 | 32 | 8 |
| Compound (b) | > 256 | > 256 | > 256 | > 256 | > 256 |
| Compound (c) | 256 | > 256 | > 256 | > 256 | |

As is demonstrated in Table 1, the compound (a) according to Example 1 offered strong antimicrobial activity. The compound (b) represented by Chemical Formula (12) and the compound (c) represented by Chemical Formula (13) were evaluated as comparative compounds. The compounds (b) and (c) are described in PTL 3, as antimicrobial compounds. The compound (b) corresponds to a compound of General Formula (2) in which R¹ is CH₂NH₂, and n is 8, except for containing acetyl groups instead of the propanoyl groups. The compound (c) corresponds to a compound of General Formula (2) in which R¹ is CH₂NH₂, and n is 8, except for containing butanoyl groups instead of the propanoyl groups.

The compound (a) and the compound (b) were evaluated for minimum inhibitory concentration by measurement under the same conditions. As a result, against *Escherichia coli,* the compound (a) had a minimum inhibitory concentration of 16 µg/mL; whereas the compound (b) had a minimum inhibitory concentration of greater than 256 µg/mL, as demonstrated in Table 1. This indicated that the compound (a) has significantly better antimicrobial activity as compared with the compounds (b) and (c). Also against the other bacteria, the compound (a) has significantly lower minimum inhibitory concentrations, as compared with the compounds (b) and (c). In particular, it is striking that the compound (a) has minimum inhibitory concentrations against *Bacillus subtilis* and *Pseudomonas aeruginosa* of 10 µg/mL or less, which level is generally considered as having strong antimicrobial activity. In the aggregate, these results demonstrate that the compound (a) is significantly improved as an antimicrobial agent, as compared with the compounds (b) and (c).

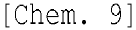

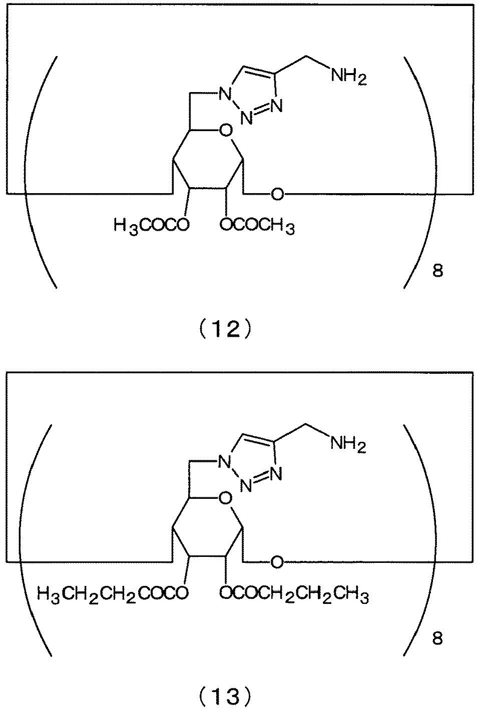

### Example 3

Aside from the above, the compound (b) was evaluated for action of enhancing the activity of another antimicrobial substance. The testing results, on the compound (b), of the action of enhancing the activity of another antimicrobial substance are given in Table 2. The compound (b) corresponds to a compound of Chemical Formula (6) in which R³ is aminomethyl; R⁴ is acetyl, and n is 8. PTL 3 fails to describe the activity enhancing action of the compound (b).

Some of existing antimicrobial substances fail to exert their inherent antimicrobial activities, due to the bacterial membrane serving as a physical barrier. In these cases, the substances offer high minimum inhibitory concentrations (MICs) necessary for inhibiting the bacterial growth and are considered as having no antimicrobial (antibacterial) activity. In particular, this allows Gram-negative bacteria, which have not only a cytoplasmic membrane, but also a bacterial outer membrane, to have remarkable drug resistance. On the basis of these, it can be rationally estimated that the combined use of an antimicrobial substance with a membrane-damaging substance allows the antimicrobial substance to offer further higher antimicrobial activity and, moreover, allows the antimicrobial substance that has been considered to have no antimicrobial activity to offer effective antimicrobial activity, where the membrane-damaging substance damages the bacterial membrane to remove the physical barrier. In the combined use, the antimicrobial substance has a lower (decreased) minimum inhibitory concentration (MIC), as compared with the case of the single use. In this testing, novobiocin or erythromycin, which is generally considered to have a low efficacy on Gram-negative bacteria, was used as the other antimicrobial substance.

The test was performed using bacteria *Escherichia coli* and *Pseudomonas aeruginosa.* Novobiocin, when used alone, had a minimum inhibitory concentration against *Escherichia coli* of 128 µg/mL, and a minimum inhibitory concentration against *Pseudomonas aeruginosa* of 2046 µg/mL. The cyclodextrin derivative (b) had a minimum inhibitory concentration against *Escherichia coli* of greater than 256 µg/mL, as presented in Table 1 and offers no antimicrobial activity at the concentration of the cyclodextrin derivative (b) in combined use in the test. In the test performed under these conditions, novobiocin, when used in combination with the cyclodextrin derivative (b), had a significantly lowered minimum inhibitory concentration and exhibited remarkable antimicrobial activity. The cyclodextrin derivative (b) allowed novobiocin to exhibit strong antimicrobial activity against *Escherichia coli,* 128 times (128/1) as much as that in single use, and, against *Pseudomonas aeruginosa,* 16 times (2048/128) as much as that in single use, where *Pseudomonas aeruginosa* generally exhibits resistance against antimicrobial agents. In addition, the cyclodextrin derivative (b) allowed erythromycin to have a significantly lowered minimum inhibitory concentration. Thus, the cyclodextrin derivative (b) exhibited remarkable antimicrobial activity enhancing action.

Specifically, against *Escherichia coli,* novobiocin has a minimum inhibitory concentration (MIC) of 128 µg/mL, and erythromycin has a minimum inhibitory concentration (MIC) of 64 µg/mL at a concentration of the cyclodextrin derivative (b) of 0 µg/mL (single use). In contrast, novobiocin has a minimum inhibitory concentration (MIC) of 1 µg/mL and erythromycin has a minimum inhibitory concentration (MIC) of 1 µg/mL at a concentration of the cyclodextrin derivative (b) of 32 µg/mL in combined use.

**[Table 2]**

| Bacterium | Concentration (in µg/mL) of cyclodextrin derivative (b) in combined use | Minimum inhibitory concentration (MIC; in µg/mL) of novobiocin | Minimum inhibitory concentration (MIC; in µg/ml) of erythromycin |
|---|---|---|---|
| *Escherichia coli* | 0 | 128 | 64 |
| | 8 | 16 | |
| | 16 | 2 | 1 |
| | 32 | 1 | 1 |
| *Pseudomonas aeruginosa* | 0 | 2048 | 256 |
| | 16 | 256 | 32 |
| | 32 | 128 | 32 |

### Example 4

Next, the compound (cyclodextrin derivative) (a) represented by Chemical Formula (11) was evaluated for antimicrobial activity enhancing action through a test performed by a procedure similar to that in Example 3. The test results are given in Table 3. The cyclodextrin derivative (a) has a minimum inhibitory concentration against *Escherichia coli* of 16 µg/mL as indicated in Table 1, and exhibits no antimicrobial activity at concentrations of the cyclodextrin derivative (a) in combined use in this test as given in Table 3. In the test under these conditions, novobiocin and erythromycin each had a significantly lowered minimum inhibitory concentration in combined use of the cyclodextrin derivative (a), and this indicates that the compound (a) exhibited remarkable antimicrobial activity enhancing action.

In contrast, the cyclodextrin derivative (c), tested as a comparative compound, did not affect novobiocin, namely, novobiocin had a non-changed inhibitory concentration of 128 µg/mL even at a concentration of the cyclodextrin derivative (c) of 32 µg/mL in combined use. This indicated that the cyclodextrin derivative (c) did not enhance or improve the antimicrobial activity. This indicates that an amino-containing structure, even when introduced, does not allow the resulting compound to exhibit or exhibit antimicrobial activity enhancing ability when the amino-containing structure and a hydrophobic moiety are out of balance. In other words, the acetyl groups or propanoyl groups replacing hydrogen atoms of secondary hydroxy groups function as hydrophobic moieties and enable the resulting compound to exhibit antimicrobial activity enhancing ability. This indicates the operations of the antimicrobial activity enhancer according to the twelfth embodiment of the present invention.

**[Table 3]**

| Bacterium | Concentration (in µg/mL) of cyclodextrin derivative (a) in combined use | Minimum inhibitory concentration (MIC; in µg/mL) of novobiocin | Minimum inhibitory concentration (MIC; in µg/mL) of erythromycin |
|---|---|---|---|
| *Escherichia coli* | 0 | 128 | 64 |
| | 2 | 8∼64 | |
| | 4 | 8∼32 | 4 |

### Example 5

In addition, a compound (d) represented by Chemical Formula (14) and a compound (e) represented by Chemical Formula (15) were synthetically prepared. The compound (d) corresponds to a compound of General Formula (2) in which R¹ is CH₂NH₂, and n is 7. The compound (e) corresponds to a compound of General Formula (2) in which R¹ is CH₂NH₂, and n is 6. The syntheses of the compounds (d) and (e) respectively employed cyclodextrin heptaazide and cyclodextrin hexaazide, instead of cyclodextrin octaazide in the synthesis of the compound (a) represented by Chemical Formula (11). The compounds (d) and (e) were evaluated for antimicrobial activity on the basis of minimum inhibitory concentrations measured by a procedure similar to that in Example 3. The results of minimum inhibitory concentration measurement are given in Table 4.

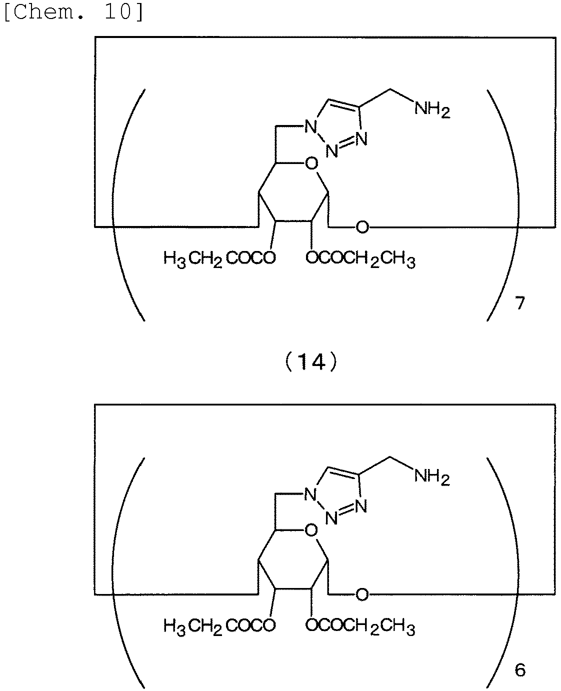

**[Table 4]**

| | Minimum Inhibitory Concentration (in µg/mL) | | | | |
|---|---|---|---|---|---|
| | *Bacillus subtilis* | *Staphylococcus aureus* | *Eschierichia coll* | *Salmonella typhimurium* | *Pseudomonas aeroginosa* |
| Compound (d) | 4 | 16 | 8 | 16 | 8 |
| Compound (e) | 8 | 16 | 8 | 16 | 8 |

As is demonstrated in Table 4, the compounds (d) and (e) exhibited strong antimicrobial activity. Accordingly, all the compounds (a), (d), and (e) have strong antimicrobial activity against not only Gram-positive bacteria, but also Gram-negative bacteria.

### Example 6

In addition, a compound (f) represented by Chemical Formula (16) was synthetically prepared. The compound (f) corresponds to a compound of General Formula (2) in which R¹ is CH₂NH₂, and n is 7, except for containing acetyl groups instead of the propanoyl groups. The compound (f) also corresponds to a compound of General Formula (6) in which R³ is CH₂NH₂, R⁴ is acetyl, and n is 7. The compound (f) was synthesized by a procedure similar to that for the compound (a) represented by Chemical Formula (11), except for using cyclodextrin heptaazide instead of cyclodextrin octaazide; and using acetic anhydride instead of propionic anhydride. The compound (f) was evaluated for action of enhancing the activity of another antimicrobial substance by a procedure similar to that in Example 4. The test results on the compound (f) for action of enhancing the antimicrobial activity of another antimicrobial substance are given in Table 5.

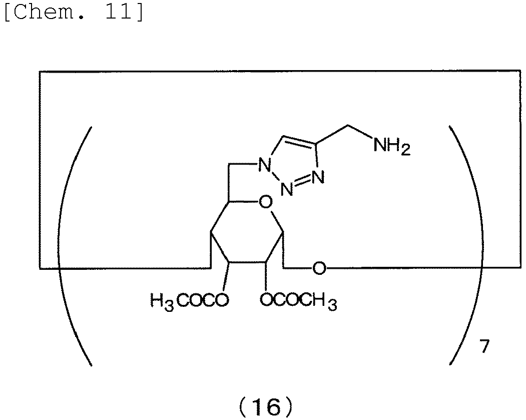

**[Table 5]**

| Bacterium | Concentration (in µg/mL) of cyclodextrin derivative (f) in combined use | Minimum inhibitory concentration (MIC; in µg/mL) of novobiocin | Minimum inhibitory concentration (MIC; in µg/mL) of erythromycin |
|---|---|---|---|
| *Escherichia coli* | 0 | 128 | 64 |
| | 8 | 16 | 16 |
| | 16 | 4 | 1 |
| | 32 | 2 | 1 |
| *Pseudomonas aeruginosa* | 0 | 2048 | 256 |
| | 16 | 256 | 64 |
| | 32 | 256 | 64 |

As is demonstrated in Table 5, novobiocin and erythromycin have remarkably lower minimum inhibitory concentrations in the combined use of the compound (f), namely, the cyclodextrin derivative (f). Thus, the compound (f) displayed remarkable antimicrobial activity enhancing action. Accordingly, both the compounds (b) and (f) have strong action as antimicrobial activity enhancers.

These results demonstrate that compounds including a cyclodextrin backbone with an amino group introduced by the click reaction and with propanoyl groups introduced on secondary hydroxy groups are effective for developing antimicrobial activity. The antimicrobial activity indicated herein is rationally assumed to be caused by the membrane damaging mechanism on the basis of their molecular design, and demonstrates the effectiveness of the molecular design to constitute, using cyclodextrins, substances that exhibit antimicrobial activity.

Herein, the antimicrobial activity against bacteria (antibacterial activity) is exhibited by linking an amino-containing structure through a triazole moiety to each glucose unit of a cyclodextrin and by further introducing propanoyl groups to the glucose unit. This indicates that antimicrobial substances can be constructed using the properties of glucose. Accordingly, it is speculated that antimicrobial substances can be constructed also by introducing such structures into oligosaccharides or polysaccharides, each of which includes glucose units as with cyclodextrins. In addition, it is rationally inferred that antimicrobial substances can be obtained from oligosaccharides and polysaccharides including monosaccharides which are glucose isomers and have similar properties, as well as from not only cyclic saccharides, but also chain saccharides, each derived from any of the monosaccharides.

The aminomethyl group was used herein as the amino-containing structure. As described above, a cyclodextrin is allowed to exhibit antimicrobial activity by introducing such an amino group by the click reaction, and further introducing propanoyl groups into the secondary hydroxy groups. Accordingly, it is rationally inferred that even another amino-containing structure than the aminomethyl group can give antimicrobial substances.

Compounds prepared by introducing an amino group into a cyclodextrin by the click reaction and further introducing acetyl groups or propanoyl groups into the secondary hydroxy groups of the cyclodextrin are effective for exhibiting the antimicrobial activity enhancing action. The performance or properties of the compounds are rationally estimated to be caused by the membrane damaging mechanism on the basis of their molecular design and demonstrate the effectiveness of the molecular design to constitute, using cyclodextrins, substances that exhibit antimicrobial activity enhancing action. Thus, it is speculated that antimicrobial activity enhancing substances can be obtained by introducing such a structure into oligosaccharides and polysaccharides, each of which includes glucose units as with cyclodextrins. In addition, it is rationally inferred that antimicrobial activity enhancing substances can be obtained from oligosaccharides and polysaccharides including units of monosaccharides which are glucose isomers and have similar properties, as well as from not only cyclic saccharides, but also chainlike saccharides, each derived from any of the monosaccharides.

The aminomethyl group was used herein as the amino-containing structure. As described above, a cyclodextrin is allowed to exhibit the antimicrobial activity enhancing action probably by introducing an amino group by the click reaction, and further introducing acetyl groups or propanoyl groups into the secondary hydroxy groups of the dextrin. Accordingly, it is rationally speculated that even another amino-containing structure than the aminomethyl group can give antimicrobial activity enhancing substances.

It is to be understood that the present invention is not limited by any of the detailed description relating to the embodiments and examples thereof; and that any and all of various modifications and variations which may readily occur to those skilled in the art should be considered to be within the scope of the present invention, without departing from the spirit and scope of the appended claims.

### Industrial Applicability

The saccharide derivatives and salts thereof according to the present invention are applicable as or in antimicrobial agents and/or antimicrobial activity enhancers.

## Claims

1. A saccharide derivative represented by one of Chemical Formulae (1) and (2), or a salt thereof, the saccharide derivative comprising units of a monosaccharide linked to each other in a chainlike or cyclic manner through glycosidic bonding and having antimicrobial action, Chemical Formulae (1) and (2) expressed as follows,
wherein R¹ represents an amino-containing structure, where R¹ is bonded to the specified triazole replacing a primary hydroxy group of the monosaccharide; the propanoyl groups each replace a hydrogen atom of a secondary hydroxy group of the monosaccharide; and n represents an integer of 3 or more:

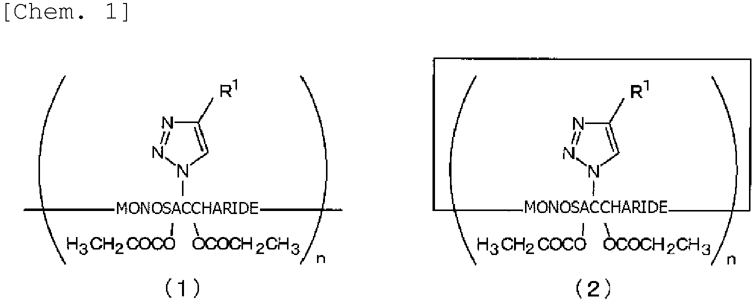

2. A saccharide derivative represented by one of Chemical Formulae (3) and (4), or a salt thereof, the saccharide derivative comprising units of a monosaccharide linked to each other in a chainlike or cyclic manner through glycosidic bonding and having antimicrobial action, Chemical Formulae (3) and (4) expressed as follows,
wherein R² represents an alkylene group, wherein R²NH₂ is bonded to the specified triazole replacing a primary hydroxy group of the monosaccharide; the propanoyl groups each replace a hydrogen atom of a secondary hydroxy group of the monosaccharide; and n represents an integer of 3 or more:

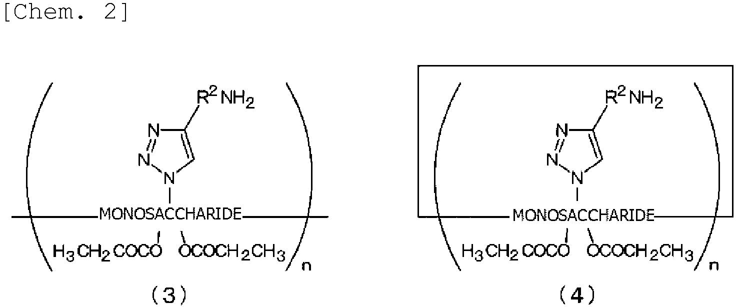

3. The saccharide derivative or a salt thereof according to claim 2,
wherein R² in one of Chemical Formulae (3) and (4) is a methylene group.

4. The saccharide derivative or a salt thereof according to any one of claims 1 to 3,
wherein the monosaccharide in the chemical formula is glucose.

5. The saccharide derivative or a salt thereof according to any one of claims 1 to 3,
wherein the monosaccharide in the chemical formula is glucose, and
wherein the saccharide derivative is a cyclic oligosaccharide comprising units of the glucose linked to each other.

6. An antimicrobial agent comprising, as an active ingredient,
the saccharide derivative or a salt thereof according to any one of claims 1 to 5.

7. A saccharide derivative represented by one of Chemical Formulae (5) and (6), or a salt thereof, the saccharide derivative comprising units of a monosaccharide linked to each other in a chainlike or cyclic manner through glycosidic bonding and having antimicrobial activity enhancing action, Chemical Formulae (5) and (6) expressed as follows,
wherein R³ represents an amino-containing structure, where R³ is bonded to the specified triazole replacing a primary hydroxy group of the monosaccharide; R⁴ is, independently in each occurrence, selected from acetyl and propanoyl and replaces a hydrogen atom of a secondary hydroxy group of the monosaccharide; and n represents an integer of 3 or more:

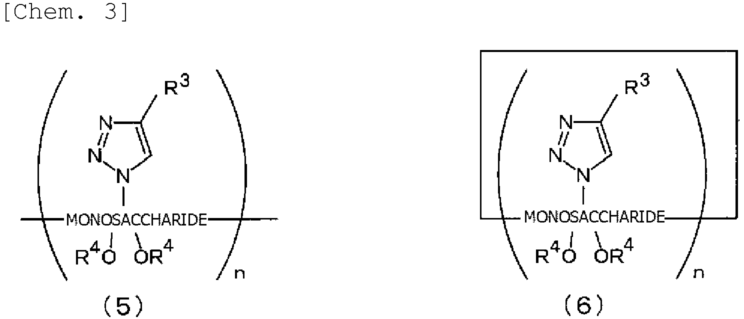

8. The saccharide derivative or a salt thereof according to claim 7,
wherein the saccharide derivative is represented by one of Chemical Formulae (7) and (8), and
the saccharide derivative comprises units of a monosaccharide linked to each other in a chainlike or cyclic manner through glycosidic bonding and has antimicrobial action and/or antimicrobial activity enhancing action, Chemical Formulae (7) and (8) expressed as follows,
wherein R⁵ represents an alkylene group, where R⁵NH₂ is bonded to the specified triazole replacing a primary hydroxy group of the monosaccharide; R⁶ is, independently in each occurrence, selected from acetyl and propanoyl and replaces a hydrogen atom of a secondary hydroxy group of the monosaccharide; and n represents an integer of 3 or more:

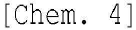

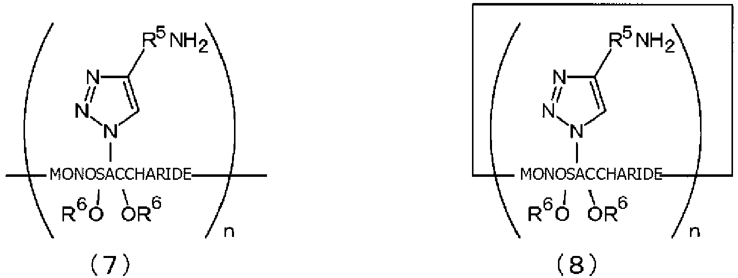

9. The saccharide derivative or a salt thereof according to claim 8,
wherein R⁵ in one of Chemical Formulae (7) and (8) is a methylene group.

10. The saccharide derivative or a salt thereof according to any one of inventions 7 to 9,
wherein the monosaccharide in the chemical formula is glucose.

11. The saccharide derivative or a salt thereof according to any one of claims 7 to 9,
wherein the monosaccharide in the chemical formula is glucose, and
wherein the saccharide derivative is an oligosaccharide comprising units of the glucose linked to each other in a cyclic manner.

12. An antimicrobial activity enhancer comprising, as an active ingredient,
the saccharide derivative or a salt thereof according to any one of claims 7 to 11.

## Amended claims

### Amended claims under Art. 19.1 PCT

2. (Amended) A saccharide derivative being represented by Chemical Formula (18) and having antimicrobial action, or a salt thereof,
wherein Chemical Formula (18) represents a cyclodextrin derivative comprising glucose units linked to each other in a cyclic manner through glycoside bonding; R² represents a methylene group; and n represents any one of 6, 7, and 8:

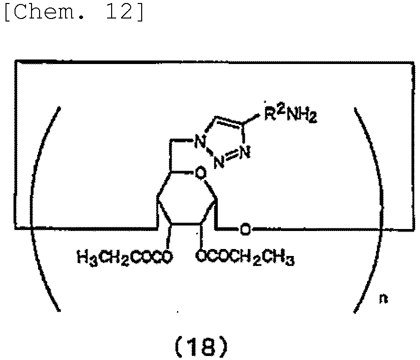

3. (Amended) The saccharide derivative or a salt thereof according to claim 2,
wherein n is 8.

6. (Amended) An antimicrobial agent comprising, as an active ingredient,
the saccharide derivative or a salt thereof according to one of claims 2 and 3.

8. (Amended) An antimicrobial activity enhancer comprising, as an active ingredient, a saccharide derivative being represented by one of Chemical Formulae (19) and (20) and having antimicrobial activity enhancing action, or a salt thereof,
wherein Chemical Formula (20) represents a cyclodextrin derivative comprising glucose units linked to each other in a cyclic manner through glycoside bonding; Chemical Formula (19) represents a saccharide derivative comprising glucose units linked to each other in a chainlike manner through glycoside bonding; R⁵ represents an alkylene group; and n represents an integer of from 5 to 100:

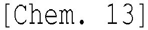

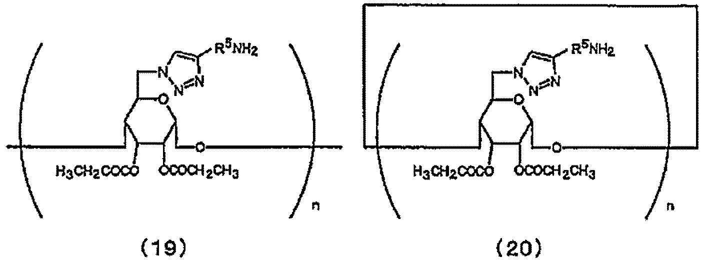

9. (Amended) The antimicrobial activity enhancer according to claim 8,
wherein the saccharide derivative is represented by Chemical Formula (20), and
wherein R⁵ is a methylene group; and n is 8.

12. (Deleted)

13. An antimicrobial activity enhancer comprising, as an active ingredient,
a saccharide derivative represented by one of Chemical Formulae (21) and (22), or a salt thereof,
wherein Chemical Formula (22) represents a cyclodextrin derivative comprising glucose units linked to each other in a cyclic manner through glycoside bonding; Chemical Formula (21) represents a saccharide derivative comprising glucose units linked to each other in a chainlike manner through glycoside bonding; R⁵ represents an alkylene group; and n represents an integer of from 5 to 100:

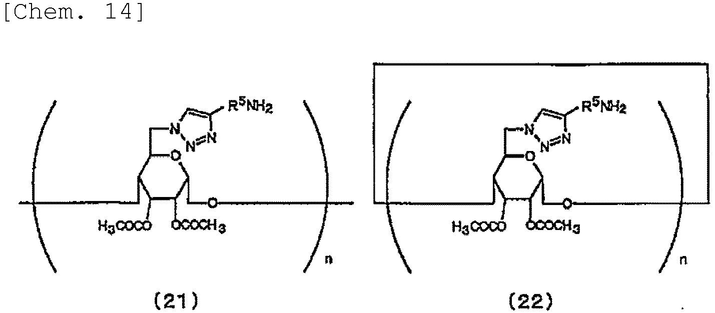

14. The antimicrobial activity enhancer according to claim 13,
wherein the saccharide derivative is represented by Chemical Formula (22), and
wherein R⁵ is a methylene group; and n is 7 or 8.

15. (Deleted)

16. (Deleted)

17. (Deleted)

18. (Amended) The antimicrobial activity enhancer according to claim 8,
wherein the saccharide derivative is represented by Chemical Formula (20).

19. (Amended) The antimicrobial activity enhancer according to one of claims 8 and 18,
wherein R⁵ is a methylene group.

20. (Amended) The antimicrobial activity enhancer according to one of claims 8 and 18,
wherein n is any one of 6, 7, and 8.

21. The antimicrobial activity enhancer according to claim 13,
wherein the saccharide derivative is represented by Chemical Formula (22).

22. The antimicrobial activity enhancer according to one of claims 13 and 21,
wherein R⁵ is a methylene group.

23. The antimicrobial activity enhancer according to one of claims 13 and 21,
wherein n is any one of 6, 7, and 8.
